# EUROPEAN PATENT SPECIFICATION

(11) **EP 2 844 344 B1**
(45) Date of publication and mention of the grant of the patent: **13.01.2021**
(21) Application number: 13784151.6
(22) Date of filing: 03.05.2013
(51) Int. Cl.: A61K 8/44, A61K 8/46, A61Q 11/00, A61K 9/00, A61P 31/18, A61P 35/00, A61K 9/08, A61P 31/02, A61P 1/02, A61P 3/10, A61P 29/00

(54) **TREATING INFLAMMATORY CONDITIONS AND IMPROVING ORAL HYGIENE USING METAL MODULATORS WITH METHYLSULFONYLMETHANE AS TRANSPORT ENHANCER**
BEHANDLUNG ENTZÜNDLICHER PROZESSE UND VERBESSERUNG DER MUNDHYGIENE UNTER VERWENDUNG VON METALLMODULATOREN MIT METHYLSULFONYLMETHAN WIE TRANSPORTVERSTÄRKER
TRAITEMENT D'ÉTATS INFLAMMATOIRES ET AMÉLIORATION DE L'HYGIÈNE BUCCALE EN UTILISANT DES MODULATEURS DE MÉTAL AVEC DU MÉTHYLSULFONYLMÉTHANE COMME ACTIVATEUR DE TRANSPORT

(30) Priority: 03.05.2012 US 201261642441 P
(43) Date of publication of application: 11.03.2015
(73) Proprietor: Bhushan, Rajiv, Mountain View, California 94041 (US); Gin, Jerry B., Sunnyvale, California 94807 (US); Goswamy, Amit, Los Gatos, California 95030 (US)
(72) Inventor: Bhushan, Rajiv, Mountain View, California 94041 (US); Gin, Jerry B., Sunnyvale, California 94807 (US); Goswamy, Amit, Los Gatos, California 95030 (US)
(74) Representative: J A Kemp LLP
(86) International application number: PCT/US2013/039573
(87) International publication number: WO 2013/166459

(56) References cited:
- WO-A2-2007/011874
- WO-A2-2008/099397
- US-A- 6 017 516
- US-A1- 2004 137 068
- US-A1- 2006 083 727
- US-A1- 2008 038 219
- US-A1- 2009 042 161
- US-A1- 2010 035 992
- US-A1- 2011 094 519
- US-B1- 6 228 347

## Description

### TECHNICAL FIELD OF THE INVENTION

This disclosure relates generally to the field of pharmacotherapy related to the treatment of disorders related to the oral cavity, dental and gingival diseases, and other adverse oral conditions. More particularly, the invention pertains to a formulation for use in a method for preventing or treating various adverse oral conditions, including those associated with dental disease. The invention pertains to the use of the formulation in improving oral health and the cosmetic appearance of tissues in the oral cavity applicable in a variety of fields, including dentistry, geriatrics, immune disorders, oncology and cosmeceuticals.

### BACKGROUND OF THE INVENTION

Biofilm, calculus and plaque are commonly known as the primary cause of dental caries gingivitis, periodontitis, mucositis and other oral conditions. Dental plaque, which exists not only on the tooth surface but also under the gums, can be defined as a diverse community of microorganisms in the form of a biofilm. The microorganisms bind tightly to one another, in addition to the solid tooth surface, by means of an extracellular matrix consisting of polymers of both host and microbial origin.

As a biofilm, dental plaque exhibits an open architecture much like that of other biofilms. The open architecture, which consists of channels and voids, helps to achieve the flow of nutrients, waste products, metabolites, enzymes, and oxygen through the biofilm. Because of this structure, a variety of microbial organisms can make up biofilms, including both aerobic and anaerobic bacteria.

Experts agree that most forms of periodontal disease are caused by specific pathogens, particularly gram-negative bacteria. The microbial composition of dental biofilms includes over 700 species of bacteria and archaea, which all exist in a relatively stable environment called microbial homeostasis. (Kroes I, Lepp PW, Reiman DA Bacterial diversity within the human subgingival crevice. Proc Natl Acad Sci USA 1999; 96(25):14547-14552.)

The recognition that dental plaque is a biofilm helps to explain why periodontal diseases have been so difficult to prevent and to treat. Periodontal pathogens within a biofilm environment behave very differently from free-floating bacteria. The protective extracellular slime matrix makes bacteria extremely resistant to antibiotics, antimicrobial agents, and host defense mechanisms.

Antibiotic doses that kill free-floating bacteria, for example, need to be increased as much as 1,500 times to kill biofilm bacteria. At these high doses, the antibiotic is more likely to kill the patient before the biofilm bacteria. (Elder MJ, at al. Biofilm-related infections in ophthalmology. Eye 1995; vol. 9 (Pt. 1):102-109.) It is likely that several mechanisms are responsible for biofilm resistance to antibiotics and antimicrobial agents. The slime layer may prevent the drugs from penetrating fully into the depth of the biofilm. Bacteria can develop resistance to antimicrobial drugs by producing a thicker protective slime layer. The slime layer may protect the bacteria against leukocytes (defensive cells of the body's immune system). Antibiotic or antimicrobial therapy usually will not kill the biofilm. Mechanical removal is the most effective treatment currently available for the control of dental plaque biofilms.

Dental plaque biofilms are responsible for many of the diseases common to the oral cavity including dental caries, periodontitis, gingivitis, and the less common peri-implantitis (similar to periodontitis, but with dental implants). However, biofilms are present on healthy teeth as well. A significant problem in the art is the cross-reactions that occur between different formulation types and/or active agents when multiple formulations with each having a different function, have to be used to treat patients with multiple oral disorders.

Therefore, there is a need for effective prophylaxis and treatment of oral conditions and disorders using formulations that eliminate cross-reactivity between different functional ingredients.

### SUMMARY OF THE INVENTION

The present invention involves the use of multifunctional formulations suitable for oral and dental therapy where at least one component of the formulation, and preferably two or more formulation components, are "multifunctional" in that they are useful in preventing or treating multiple conditions and disorders, or have more than one mechanism of action, or both.

The present invention therefore provides an oral formulation for use in a method for preventing or treating an adverse oral condition in a mammalian individual susceptible to or afflicted with said adverse oral condition, said oral formulation being an oral solution, suspension, paste or gel, and comprising:
a biocompatible chelating agent at a concentration of at least 0.1% by weight;
an effective concentration of a permeation enhancer which is methylsulfonylmethane; and
a pharmaceutically acceptable carrier which is at least partially aqueous;
wherein either (i) the formulation is a hydrogel and carrier comprises a swellable, gel-forming polymer, or (ii) the formulation is able to form a hydrogel in situ following administration, and comprises a thermo-reversible hydrogel-forming polymer; and
wherein said method comprises topically administering the formulation to any part of the oral cavity of the individual.

Also provided is an oral delivery system for use in a method for preventing or treating an adverse oral condition in a mammalian individual susceptible to or afflicted with said adverse oral condition, said oral delivery system comprising a liposomal dispersion of the formulation for use of the present invention,
wherein said method comprises topically administering the formulation to any part of the oral cavity of the individual.

Additionally provided is a sterile insert for delivery of a formulation to the oral cavity, comprising:
a controlled release implant housing the formulation for use the present invention and suitable for implantation into any part of the oral cavity,
wherein the implant is comprised of a polymeric matrix that is configured to gradually release the formulation to the oral tissues through dissolution of the matrix and/or diffusion, and that which is completely soluble and/or biodegradable in the oral tissues,
wherein the implant comprises a reservoir housing the formulation and enclosed in a polymeric membrane through which the formulation gradually diffuses.

In some embodiments, the adverse oral conditions include gingivitis, periodontal disease, removal of calculus to improve dental hygiene, and control of dental plaque and biofilm.

In further embodiments, the adverse oral conditions include inflammation and oxidative and/or free radical damage within the oral cavity are provided. Treatable conditions may relate to other conditions or diseases, including diabetes, AIDS and cancer.

The method involves administering to the subject an effective amount of a formulation composed of a therapeutically effective amount of a chelating agent and an effective transport-enhancing amount of a transport enhancer which is methylsulfonylmethane (also referred to as methylsulfone, dimethylsulfone, and DMSO₂).

The transport enhancer is present in an amount effective to facilitate transport of the chelating agent such that the chelating agent is delivered in an amount effective to treat an adverse oral condition.

The chelating agent can be ethylene diamine tetra-acetic acid (EDTA) and the like.

The oral formulation may be administered in any form suitable for oral administration, e.g., as a solution, suspension, paste, ointment, gel, liposomal dispersion, colloidal micro-particle suspension, or the like, or in an oral insert, e.g., in an optionally biodegradable controlled release polymeric matrix. Significantly, at least one component of the formulation, and preferably two or more formulation components, is "multifunctional" in that it is useful in preventing or treating multiple conditions and disorders, or have more than one mechanism of action, or both. Accordingly, the present formulations eliminate a significant problem in the art, namely, cross-reaction between different formulation types and/or active agents when multiple formulations are used to treat a patient with multiple oral disorders. Additionally, in a preferred embodiment, the formulation is entirely composed of components that are naturally occurring and/or as GRAS ("Generally Regarded as Safe") by the U.S. Food and Drug Administration.

The adverse oral conditions generally, although not necessarily, involve oxidative and/or free radical damage in the oral cavity, and include, by way of example, conditions, diseases, or disorders of the oral cavity.

### BRIEF DESCRIPTION OF THE DRAWINGS

The following drawings form part of the present specification and are included to further demonstrate certain aspects of the present disclosure, the inventions of which can be better understood by reference to one or more of these drawings in combination with the detailed description of specific embodiments presented herein.

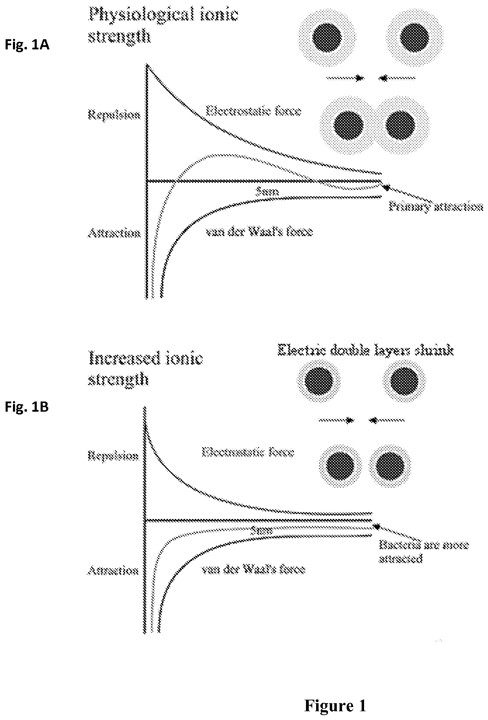

Figure 1A shows attraction of bacterial species to the tooth under physiologic ionic strength. Figure 1B shows increased attraction of bacterial species to the tooth under increased ionic strength.

### DETAILED DESCRIPTION OF THE INVENTION

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

As used herein and in the appended claims, the singular forms "a," "and," and "the" include plural referents unless the context clearly dictates otherwise. Thus, for example, "a transport enhancer" encompasses a plurality of transport enhancers as well as a single transport enhancer. Reference to "a chelating agent" includes reference to two or more chelating agents as well as a single chelating agent, and so forth. In this specification and in the claims that follow, reference will be made to a number of terms, which shall be defined to have the following meanings:

When referring to a formulation component, it is intended that the term used, e.g., "agent," encompass not only the specified molecular entity but also its pharmaceutically acceptable analogs, including, but not limited to, salts, esters, amides, prodrugs, conjugates, active metabolites, and other such derivatives, analogs, and related compounds.

The terms "treating" and "treatment" as used herein refer to the administration of an agent or formulation to a clinically symptomatic individual afflicted with an adverse condition, disorder, or disease, so as to effect a reduction in severity and/or frequency of symptoms, eliminate the symptoms and/or their underlying cause, and/or facilitate improvement or remediation of damage. The terms "preventing" and "prevention" refer to the administration of an agent or composition to a clinically asymptomatic individual who is susceptible to a particular adverse condition, disorder, or disease, and thus relates to the prevention of the occurrence of symptoms and/or their underlying cause. Unless otherwise indicated herein, either explicitly or by implication, if the term "treatment" (or "treating") is used without reference to possible prevention, it is intended that prevention be encompassed as well, such that "a method for the treatment of gingivitis" would be interpreted as encompassing "a method for the prevention of gingivitis."

"Optional" or "optionally present" - as in an "optional substituent" or an "optionally present additive" means that the subsequently described component (e.g., substituent or additive) may or may not be present, so that the description includes instances where the component is present and instances where it is not.

By "pharmaceutically acceptable" is meant a material that is not biologically or otherwise undesirable, e.g., the material may be incorporated into a formulation of the invention without causing any undesirable biological effects or interacting in a deleterious manner with any of the other components of the dosage form formulation. However, when the term "pharmaceutically acceptable" is used to refer to a pharmaceutical excipient, it is implied that the excipient has met the required standards of toxicological and manufacturing testing and/or that it is included on the Inactive Ingredient Guide prepared by the U.S. Food and Drug Administration. As explained in further detail infra, "pharmacologically active" (or simply "active") as in a "pharmacologically active" derivative or analog refers to derivative or analog having the same type of pharmacological activity as the parent agent. The terms "treating" and "treatment" as used herein refer to reduction in severity and/or frequency of symptoms, elimination of symptoms and/or underlying cause, prevention of the occurrence of symptoms and/or their underlying cause, and improvement or remediation of an undesirable condition or damage. Thus, for example, "treating" a subject involves prevention of an adverse condition in a susceptible individual as well as treatment of a clinically symptomatic individual by inhibiting or causing regression of the condition. The term "chelating agent" (or "active agent") refers to any chemical compound, complex or composition that exhibits a desirable effect in the biological context, i.e., when administered to a subject or introduced into cells or tissues in vitro. The term includes pharmaceutically acceptable derivatives of those active agents specifically mentioned herein, including, but not limited to, salts, esters, amides, prodrugs, active metabolites, isomers, analogs, crystalline forms, hydrates, and the like. When the term "chelating agent" is used, or when a particular chelating agent is specifically identified, it is to be understood that pharmaceutically acceptable salts, esters, amides, prodrugs, active metabolites, isomers, analogs, etc. of the agent are intended as well as the agent per se.

By an "effective" amount or a "therapeutically effective" amount of an active agent is meant a nontoxic but sufficient amount of the agent to provide a beneficial effect. The amount of active agent that is "effective" will vary from subject to subject, depending on the age and general condition of the individual, the particular active agent or agents, and the like. Unless otherwise indicated, the term "therapeutically effective" amount as used herein is intended to encompass an amount effective for the prevention of an adverse condition and/or the amelioration of an adverse condition, i.e., in addition to an amount effective for the treatment of an adverse condition.

The term "controlled release" refers to an agent-containing formulation or fraction thereof in which release of the agent is not immediate, i.e., with a "controlled release" formulation, administration does not result in immediate release of the agent into an absorption pool. The term is used interchangeably with "nonimmediate release" as defined in Remington: The Science and Practice of pharmacy, Nineteenth Ed. (Easton, Pa.: Mack Publishing Company, 1995). In general, the term "controlled release" as used herein refers to "sustained release" rather than to "delayed release" formulations. The term "sustained release" (synonymous with "extended release") is used in its conventional sense to refer to a formulation that provides for gradual release of an agent over an extended period of time.

An adverse oral condition as that term is used herein may be a "normal" condition that is frequently seen in individuals (e.g., increased dental calculus) or a pathologic condition that may or may not be associated with a named disease. The latter adverse oral conditions include a wide variety of dental disorders and diseases, associated with deposition of mineral deposits, biofilm build-up, infections and inflammation. It should also be emphasized that the present formulation can be advantageously employed to improve oral health, in general, in any mammalian individual.

As will be apparent to those of skill in the art upon reading this invention, each of the individual embodiments described and illustrated herein has discrete components and features which may be readily separated from or combined with the features of any of the other several embodiments. Any recited method can be carried out in the order of events recited or in any other order that is logically possible.

Unless otherwise indicated, the invention is not limited to specific formulation components, modes of administration, chelating agents, manufacturing processes, or the like, as such may vary.

Unless otherwise defined, all technical and scientific terms used herein have the same meaning as commonly understood by one of ordinary skill in the art to which this invention pertains. In the case of conflict, the present document, including definitions will control.

### Definitions

Chelating agent: Chelation is a chemical combination with a metal in complexes in which the metal is part of a ring. An organic ligand is called a chelator or chelating agent, the chelate is a metal complex. The larger number of ring closures to a metal atom the more stable is the compound. The stability of a chelate is also related to the number of atoms in the chelate ring. Monodentate ligands which have one coordinating atom like H₂O or NH₃ are easily broken apart by other chemical processes, whereas polydentate chelators, donating multiple binds to metal ion, provide more stable complexes. Chlorophyll, a green plant pigment, is a chelate that consists of a central magnesium atom joined with four complex chelating agent (pyrrole ring). Heme is an iron chelate which contains iron (II) ion in the center of the porphyrin. Chelating agents offers a wide range of sequestrants to control metal ions in aqueous systems. By forming stable water soluble complexes with multivalent metal ions, chelating agents prevent undesired interaction by blocking normal reactivity of metal ions. EDTA (ethylenediamine tetraacetate) is a good example of common chelating agents which have nitrogen atoms and short chain carboxylic groups.

Examples of chelators of iron and calcium include, but are not limited to, Diethylene triamine pentaacetic acid (DTPA), ethylene diamine tetraacetic acid (EDTA), nitrilotriacetic acid (NTA), 1,3-propylene diamine tetraacetic acid (PDTA), Ethylene diamine disuccinic acid (EDDS), and ethylene glycol tetraacetic acid (EGTA). Any suitable chelating agent known in the art, which is biologically safe and able to chelate iron, calcium or other metals, is suitable for the invention.

Compounds useful as chelating agents herein include any compounds that coordinate to or form complexes with a divalent or polyvalent metal cation, thus serving as a sequestrant of such cations. Accordingly, the term "chelating agent" herein includes not only divalent and polyvalent ligands (which are typically referred to as "chelators") but also monovalent ligands capable of coordinating to or forming complexes with the metal cation.

The biocompatible chelating agent is a sequestrant of divalent or polyvalent metal cations, and generally represents about 0.1 wt. % to 15 wt. %, about 0.6 wt. % to 10 wt. %, or preferably about 1.0 wt. % to 5.0 wt. %, of the formulation. The invention is not limited with regard to specific biocompatible chelating agents, and any biocompatible chelating agent can be used providing that it is capable of being buffered to a pH in the range of about 4.5 to about 9.0 and does not interact with any other component of the formulation. Suitable biocompatible chelating agents useful in conjunction with the present invention include, without limitation, monomeric polyacids such as EDTA, cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, acceptable salts thereof, and combinations of any of the foregoing. Other exemplary chelating agents include: phosphates, e.g., pyrophosphates, tripolyphosphates, and, hexametaphosphates; chelating antibiotics such as chloroquine and tetracycline; nitrogen-containing chelating agents containing two or more chelating nitrogen atoms within an imino group or in an aromatic ring (e.g., diimines, 2,2'-bipyridines, etc.); and polyamines such as cyclam (1,4,7,11-tetraazacyclotetradecane), N--(C₁-C₃₀ alkyl)-substituted cyclams (e.g., hexadecyclam, tetramethylhexadecylcycla- m), diethylenetriamine (DETA), spermine, diethylnorspermine (DENSPM), diethylhomo-spermine (DEHOP), and deferoxamine (N'-[5-[[4-[[5-(acetylhydroxyamino)pentyl]amino]-1,4-dioxobutyl]hydroxyamino]pcntyl]-N'-(5-aminopent-yl)-N-hydroxybutanediamide; also known as desferrioxamine B and DFO).

Suitable biocompatible chelating agents useful in conjunction with the present invention include, without limitation, monomeric polyacids such as EDTA, cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, pharmaceutically acceptable salts thereof, and combinations of any of the foregoing. Other exemplary chelating agents include: phosphates, e.g., pyrophosphates, tripolyphosphates, and hexametaphosphates.

EDTA and ophthalmologically acceptable EDTA salts are particularly preferred, wherein representative ophthalmologically acceptable EDTA salts are typically selected from diammonium EDTA, disodium EDTA, dipotassium EDTA, triammonium EDTA, trisodium EDTA, tripotassium EDTA, and calcium disodium EDTA.

EDTA has been widely used as an agent for chelating metals in biological tissue and blood, and has been suggested for inclusion in various formulations. For example, U.S. Pat. No. 6,348,508 to Denick Jr. et al. describes EDTA as a sequestering agent to bind metal ions. In addition to its use as a chelating agent, EDTA has also been widely used as a preservative in place of benzalkonium chloride, as described, for example, in U.S. Pat. No. 6,211,238 to Castillo et al. U.S. Pat. No. 6,265,444 to Bowman et al. discloses use of EDTA as a preservative and stabilizer. However, EDTA has generally not been applied topically in any significant concentration formulations because of its poor penetration across biological membranes and biofilms including skin, cell membranes and even biofilms like dental plaque.

In some embodiments, the chelating agent incorporated in the formulation is a prochelator. A prochelator is any molecule that is converted to a chelator when exposed to the appropriate chemical or physical conditions. For example, BSIH (isonicotinic acid [2-(4,4,5,5-tetramethyl-[1,3,2]dioxaborolan-2-yl)-benzylidene]-hydrazide) prochelators are converted by hydrogen peroxide into SIH (salicylaldehyde isonicotinoyl hydrazone) iron-chelating agents that inhibit iron-catalyzed hydroxyl radical generation.

The inactivated metal ion sequestering agent is sometimes referred to herein as a "prochelator," although sequestration of metal ions can involve sequestration and complexation processes beyond the scope of chelation per se. The term "prochelator" is analogous to the term "prodrug" insofar as a prodrug is a therapeutically inactive agent until activated in vivo, and the prochelator, as well, is incapable of sequestering metal ions until activated in vivo.

Transport Enhancer: The transport enhancer is selected to facilitate the transport of a chelating agent through the tissues, extra-cellular matrices, and/or cell membranes of a body. An "effective amount" of the transport enhancer represents an amount and concentration within a formulation of the invention that is sufficient to provide a measurable increase in the penetration of a chelating agent through one or more of the sites of oral cavity or teeth in a subject than would otherwise be the case without the inclusion of the transport enhancer within the formulation.

In certain instances, the transport enhancer may be present in a formulation of the invention in an amount that ranges from about 0.01 wt.% or less to about 30 wt.% or more, typically in the range of about 0.1 wt.% to about 20 wt.%, more typically in the range of about 1 wt.% to about 11 wt.%, and most typically in the range of about 2 wt.% to about 8 wt.%, for instance, 5 wt.%.

The transport enhancer is methylsulfonylmethane (MSM).

The phrase "having the formula" or "having the structure" is not intended to be limiting and is used in the same way that the term "comprising" is commonly used. The term "alkyl" refers to a linear, branched, or cyclic saturated hydrocarbon group containing 1 to 6 carbon atoms, such as methyl, ethyl, n-propyl, isopropyl, n-butyl, isobutyl, t-butyl, cyclopentyl, cyclohexyl and the like. If not otherwise indicated, the term "alkyl" includes unsubstituted and substituted alkyl, wherein the substituents may be, for example, halo, hydroxyl, sulfhydryl, alkoxy, acyl, etc. The term "alkoxy" intends an alkyl group bound through a single, terminal ether linkage; that is, an "alkoxy" group may be represented as -O-alkyl where alkyl is as defined above. The term "aryl" refers to an aromatic substituent containing a single aromatic ring or multiple aromatic rings that are fused together, directly linked, or indirectly linked (such that the different aromatic rings are bound to a common group such as a methylene or ethylene moiety). Preferred aryl groups contain 5 to 14 carbon atoms. Exemplary aryl groups are contain one aromatic ring or two fused or linked aromatic rings, e.g., phenyl, naphthyl, biphenyl, diphenylether, diphenylamine, benzophenone, and the like. "Aryl" includes unsubstituted and substituted aryl, wherein the substituents may be as set forth above with respect to optionally substituted "alkyl" groups. The term "aralkyl" refers to an alkyl group with an aryl substituent, wherein "aryl" and "alkyl" are as defined above. Preferred aralkyl groups contain 6 to 14 carbon atoms, and particularly preferred aralkyl groups contain 6 to 8 carbon atoms. Examples of aralkyl groups include, without limitation, benzyl, 2-phenyl-ethyl, 3 -phenylpropyl, 4-phenyl-butyl, 5 -phenyl -pentyl, 4-phenylcyclohexyl, 4-benzylcyclohexyl, 4-phenylcyclohexylmethyl, 4-benzylcyclohexylmethyl, and the like. The term "acyl" refers to substituents having the formula -(CO)-alkyl, -(CO)-aryl, or -(CO)-aralkyl, wherein "alkyl," "aryl, and "aralkyl" are as defined above.

### Treating Dental Plaque Biofilms

The formation of dental plaque biofilms includes a series of steps that begins with the initial colonization of the pellicle and ends with the complex formation of a mature biofilm. Dental plaque biofilms exist on a variety of tooth surfaces including fissures, smooth surfaces and gingival crevices, however they are most likely to be seen in their mature state in the more stagnant sites, like fissures and crevices, as these places provide protection from the forces of removal, like a toothbrush. Additionally, through the growth process of the plaque biofilm, the microbial composition changes from one that is primarily gram-positive and streptococcus-rich to a structure filled with gram-negative anaerobes in its more mature state.

The first step in plaque biofilm development is the adsorption of host and bacterial molecules to the tooth surface. Within minutes of tooth eruption or a cleaning, pellicle formation begins, which can be defined as a thin coat of salivary proteins. The pellicle acts like an adhesive by sticking to the tooth surface and encouraging a conditioning film of bacteria to attach to the pellicle. This conditioning film directly influences the initial microbial colonization, and continues to adsorb bacteria to the tooth surface.

Healthy tooth surfaces and gingivae tend to only be associated with this first phase of biofilm development. It consists of an initial few layers (1-20) of mostly gram-positive cocci bacteria, followed by some gram-positive rods and fillaments and a very small amount of gram-negative cocci.

The mouth comprises a number of quite distinct habitats most of which are bathed in saliva. In order to survive in the mouth bacteria must attach to one of its surfaces or risk being swallowed. Bacteria attaching to exposed smooth surfaces in the mouth must be quite firmly attached to resist the flow of saliva. Any build-up of cells due to multiplication is more easily dislodged because the mass of bacteria experiences a greater shear force. This does not mean that the exposed, smooth, surfaces of teeth are devoid of attached bacteria because some species have evolved efficient adhesion mechanisms. It does mean, however, that any significant build-up is inhibited and that plaque accumulation is limited to sheltered sites such as interproximal areas, the gingival margin and fissures. Bacteria will also accumulate in defects.

Before plaque can accumulate, the tooth has to be colonized by bacteria which then multiply and attract further colonizers. These "first colonizers" are known as pioneer species and, in the mouth comprise: (a) *Streptococcus oralis*; (b) *Streptococcus mitis*; and (c) *Streptococcus sanguis.*

The surfaces of these cells and, in fact the surfaces of nearly all cells, are negatively charged because of the presence of proteins and other wall and cell membrane components which contain phosphate, carboxyl and other acidic groups. Furthermore, nearly all non-biological surfaces are also negatively charged. Sometimes this is due to the accumulation of organic material which adsorbs to the surface from the environment and sometimes because the surface is inherently negatively charged because of its chemistry. However, the presence of high amounts of positively charged ionic calcium in both the saliva, and in the plaque fluid, causes the bacteria to be attracted to the negatively charged surface.

In accordance with the Derjaguin and Landau, Verwey and Overbeek (DLVO) theory on the causes of precipitation of colloidal particles (Derjaguin, B.; Landau, L. (1941) Acta Physico Chemica URSS 14: 633; Verwey, E. J. W.; Overbeek, J. Th. G. (1948), Theory of the stability of lyophobic colloids, Amsterdam: Elsevier), both electrostatic forces of attraction and repulsion, as well as the attractive van der Waal's forces play a key role in causing the migration of bacteria to the surface of the teeth. Increasing the concentration of ionic calcium in the plaque fluid causes the electric double layer surrounding the microbes to shrink. This reduces the electrostatic repulsive forces, and enables the bacteria to come into the domain of the much stronger van der Waal forces. This can be seen in Figures 1A and IB.

As the concentration of calcium continues to build in the plaque, it reaches levels, where small changes in pH can cause the precipitation of the calcium phosphate onto the surface in the form of brushite, the major component of dental calculus. These precipitates build up over time both sub and supra gingivally. This deposit will then injure and damage the gingivae, leading to inflammation and subsequently gingivitis.

Since calcium is involved in plaque production, calculus production, and in the causation of inflammation, a reduction in calcium levels will play a key role in treating the adverse conditions in the oral cavity. Current treatment modalities do not take this approach, but rather depend upon mechanical removal of plaque and calculus, and there is an attempt to control the inflammation by means of steroids or NSAIDs.

Removal of calcium could be accomplished by means of calcium chelators. However chelators are also negatively charged molecules, and are therefore repelled from the plaque surface. Therefore to accomplish the task of getting these chelators into the plaque and close to the calcium, a charge masking, permeation enhancing carrier would allow the chelators to get to the target metal ion, e.g. calcium. The sequestration inactivating moiety may also facilitate transport of the metal ion sequestering agent through biological membranes.

Without wishing to be bound by theory, it appears that a significant role played by the biocompatible chelating agent in the present formulations is in the removal of the calcium from the dental plaque and will allow for easier mechanical removal, and slow down the rebuilding of the unhealthy plaque. In addition, by chelating metal ions such as copper, iron, and calcium, which are critical to the formation and proliferation of free radicals in the oral tissue, the chelating agent forms complexes that are flushed into the bloodstream and excreted renally. In this way, the production of oxygen free radicals and reactive molecular fragments is reduced, in turn reducing pathological lipid peroxidation of cell membranes, DNA, enzymes, and lipoproteins, allowing the body's natural healing mechanisms to halt and reverse disease processes in progress.

Accordingly, the chelating agent is multifunctional in the context of the present invention, insofar as the agent serves to decrease unwanted proteinase (e.g., collagenase) activity, prevent formation of mineral deposits, and/or reduce mineral deposits that have already formed, and reduce calcification, in addition to acting as a preservative and stabilizing agent. The formulation also includes an effective amount of methylsulfonylmethane (MSM; also referred to as methyl sulfone) as a permeation enhancer that facilitates penetration of the formulation components through cell membranes, tissues, and extracellular matrices, including the gums and other oral tissue. The "effective amount" of the permeation enhancer represents a concentration that is sufficient to provide a measurable increase in penetration of one or more of the formulation components through membranes, tissues, and extracellular matrices as just described.

MSM is an odorless, highly water-soluble (34% w/v @ 79° F.) white crystalline compound with a melting point of 108-110° C. and a molecular weight of 94.1 g/mol. MSM serves as a multifunctional agent herein, insofar as the agent not only increases cell membrane permeability, but also acts as a "transport facilitating agent" (TFA) that aids in the transport of one or more formulation components to oral tissues. Furthermore, MSM per se provides medicative effects, and can serve as an anti-inflammatory agent as well as an analgesic. MSM also acts to improve oxidative metabolism in biological tissues, and is a source of organic sulfur, which assists in the reduction of scarring. MSM additionally possesses unique and beneficial solubilization properties, in that it is soluble in water, as noted above, but exhibits both hydrophilic and hydrophobic properties because of the presence of polar S=O groups and nonpolar methyl groups. The molecular structure of MSM also allows for hydrogen bonding with other molecules, i.e., between the oxygen atom of each S=O group and hydrogen atoms of other molecules, and for formation of van der Waal associations, i.e., between the methyl groups and nonpolar (e.g., hydrocarbyl) segments of other molecules. Ideally, the concentration of MSM in the present formulations is in the range of about 0.1 wt. % to 40 wt. %, or from about 1 wt.% to about 4, 5, 6, 7, 8, 10, 15 wt.%, and preferably between about 1.5 wt. % to 8.0 wt. %.

Other optional additives in the present formulations include secondary enhancers, i.e., one or more additional permeation enhancers. For example, formulation of the invention can contain added DMSO. Since MSM is a metabolite of DMSO (i.e., DMSO is enzymatically converted to MSM), incorporating DMSO into an MSM-containing formulation of the invention will tend to gradually increase the fraction of MSM in the formulation. DMSO also serves as a free radical scavenger, thereby reducing the potential for oxidative damage. If DMSO is added as a secondary enhancer, the amount is preferably in the range of about 1.0 wt. % to 2.0 wt. % of the formulation, and the weight ratio of MSM to DMSO is typically in the range of about 1:50 to about 50:1.

The formulations of the invention are useful in treating a wide variety of adverse oral conditions, including gingivitis, periodontal disease, dental caries and cavities, mouth sores, and all kinds of oral inflammation. It is also useful for treating oral plaque and dental calculus.

### Formulations

A variety of means can be used to formulate the compositions of the invention. Techniques for formulation and administration may be found in "Remington: The Science and Practice of Pharmacy," Twentieth Edition, Lippincott Williams & Wilkins, Philadelphia, PA (1995). For human or animal administration, preparations should meet sterility, pyrogenicity, general safety and purity standards comparable to those required by the FDA. Administration of the pharmaceutical formulation can be performed in a variety of ways, as described herein.

Other possible additives for incorporation into the formulations that are at least partially aqueous include, without limitation, thickeners, isotonic agents, buffering agents, and preservatives, providing that any such excipients do not interact in an adverse manner with any of the formulation's other components. It should also be noted that preservatives are not generally necessarily in light of the fact that the selected chelating agent itself serves as a preservative. Suitable thickeners will be known to those of ordinary skill in the art of formulation, and include, by way of example, cellulosic polymers such as methylcellulose (MC), hydroxyethylcellulose (HEC), hydroxypropylcellulose (HPC), hydroxypropylmethylcellulose (HPMC), and sodium carboxymethylcellulose (NaCMC), and other swellable hydrophilic polymers such as polyvinyl alcohol (PVA), hyaluronic acid or a salt thereof (e.g., sodium hyaluronate), and crosslinked acrylic acid polymers commonly referred to as "carbomers" (and available from B.F. Goodrich as Carbopol® polymers). Various organic gums such as but not limited to Xanthan gum and Konjac gum. The preferred amount of any thickener is such that a viscosity above 10,000 cps is provided, as a gel having a viscosity above this figure generally considered optimal for both comfort and retention of the formulation on the oral tissues. Any suitable isotonic agents and buffering agents commonly used in oral formulations may be used, providing the pH of the formulation is maintained in the range of about 4.5 to about 9.0, preferably in the range of about 6.8 to about 7.8, and optimally at a pH of about 7.4.

The formulations of the invention also include a pharmaceutically acceptable carrier, which will depend on the particular type of formulation. The formulations for use of the invention are provided as an oral solution, suspension, paste or gel, and the carrier is at least partially aqueous.

The pharmaceutical formulation may be a solid, semi-solid or liquid, such as, for example, a liquid, a cream, a suspension, an emulsion, beads, a powder, or the like, preferably in unit dosage form suitable for single administration of a precise dosage. Suitable pharmaceutical formulations and dosage forms may be prepared using conventional methods known to those in the field of pharmaceutical formulation and described in the pertinent texts and literature, e.g., in Remington: The Science and Practice of Pharmacy, cited previously herein.

The formulations of the invention may also be prepared as a hydrogel, dispersion, or colloidal suspension. Hydrogels are formed by incorporation of a swellable, gel-forming polymer such as those set forth above as suitable thickening agents (i.e., MC, HEC, HPC, HPMC, NaCMC, PVA, or hyaluronic acid or a salt thereof, e.g., sodium hyaluronate), except that a formulation referred to in the art as a "hydrogel" typically has a higher viscosity than a formulation referred to as a "thickened" solution or suspension. In contrast to such preformed hydrogels, a formulation may also be prepared so as to form a hydrogel in situ following application into the oral cavity. Such gels are liquid at room temperature but gel at higher temperatures (and thus termed "thermoreversible" hydrogels), such as when placed in contact with body fluids. Biocompatible polymers that impart this property include acrylic acid polymers and copolymers, N-isopropylacrylamide derivatives, and ABA block copolymers of ethylene oxide and propylene oxide (conventionally referred to as "poloxamers" and available under the Pluronic® trade name from BASF-Wyandotte). The formulations can also be prepared in the form of a dispersion or colloidal suspension. Preferred dispersions are liposomal, in which case the formulation is enclosed within "liposomes," microscopic vesicles composed of alternating aqueous compartments and lipid bilayers. Colloidal suspensions are generally formed from microparticles, i.e., from microspheres, nanospheres, microcapsules, or nanocapsules, wherein microspheres and nanospheres are generally monolithic particles of a polymer matrix in which the formulation is trapped, adsorbed, or otherwise contained, while with microcapsules and nanocapsules, the formulation is actually encapsulated. The upper limit for the size for these microparticles is about 5µ to about 10µ.

The formulations may also be incorporated into a sterile oral insert that provides for controlled release of the formulation over an extended time period, generally in the range of about 12 hours to 60 days, and possibly up to 12 months or more, following implantation of the insert into any tissue of the of the oral cavity. One type of oral insert is an implant in the form of a monolithic polymer matrix that gradually releases the formulation to the oral tissues through diffusion and/or matrix degradation. With such an insert, it is preferred that the polymer be completely soluble and or biodegradable (i.e., physically or enzymatically eroded in the tissues) so that removal of the insert is unnecessary. These types of inserts are well known in the art, and are typically composed of a water-swellable, gel-forming polymer such as collagen, polyvinyl alcohol, or a cellulosic polymer. Another type of insert that can be used to deliver the present formulation is a diffusional implant in which the formulation is contained in a central reservoir enclosed within a permeable polymer membrane that allows for gradual diffusion of the formulation out of the implant. Osmotic inserts may also be used, i.e., implants in which the formulation is released as a result of an increase in osmotic pressure within the implant following application to the oral tissue and subsequent absorption.

The chelating agent may be administered, if desired, in the form of a salt, ester, crystalline form, hydrate, or the like, provided it is pharmaceutically acceptable. Salts, esters, etc. may be prepared using standard procedures known to those skilled in the art of synthetic organic chemistry and described, for example, by J. March, Advanced Organic Chemistry: Reactions, Mechanisms and Structure, 4th Ed. (New York: Wiley- Interscience, 1992).

The amount of chelating agent administered will depend on a number of factors and will vary from subject to subject and depend on the particular chelating agent, the particular disorder or condition being treated, the severity of the symptoms, the subject's age, weight and general condition, and the judgment of the prescribing physician. The term "dosage form" denotes any form of a pharmaceutical composition that contains an amount of chelating agent and transport enhancer sufficient to achieve a therapeutic effect with a single administration or multiple administrations. The frequency of administration that will provide the most effective results in an efficient manner without overdosing will vary with the characteristics of the particular active agent, including both its pharmacological characteristics and its physical characteristics, such as hydrophilicity.

The oral formulations may also include conventional additives such as opacifiers, flavoring agents, antioxidants, fragrance, colorant, gelling agents, thickening agents, stabilizers, surfactants, and the like. Other agents may also be added, such as antimicrobial agents, to prevent spoilage upon storage, i.e., to inhibit growth of microbes such as yeasts and molds. Suitable antimicrobial agents are typically selected from the methyl and propyl esters of p-hydroxybenzoic acid (i.e., methyl and propyl paraben), sodium benzoate, sorbic acid, imidurea, and combinations thereof.

The dosage regimen will depend on a number of factors that may readily be determined, such as severity of the condition and responsiveness of the condition to be treated, but will normally be one or more doses per day, with a course of treatment lasting from a single dose to multiple doses over a day or several days to several months, or until a cure is effected or a diminution of disease state or other adverse condition is achieved.

### EXAMPLES

The following examples are put forth so as to provide those skilled in the art with a complete invention and description of how to make and use embodiments in accordance with the invention, and are not intended to limit the scope of what the inventors regard as their discovery. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1: Preparation of Oral Formulation A

Lotions comprising the formulations were prepared using EDTA (tetrasodium salt) and MSM, which were purchased from Sigma. All examples used Oral Formulation A, which contained 2.6% EDTA and 5.4% MSM.

Effectiveness of Lotion A is tested on subjects suffering from adverse oral conditions, including gingivitis, periodontal disease, dental caries and cavities, mouth sores, and all kinds of oral inflammation.

## Claims

1. An oral formulation for use in a method for preventing or treating an adverse oral condition in a mammalian individual susceptible to or afflicted with said adverse oral condition, said oral formulation being an oral solution, suspension, paste or gel, and comprising:
a biocompatible chelating agent at a concentration of at least 0.1% by weight;
an effective concentration of a permeation enhancer which is methylsulfonylmethane; and
a pharmaceutically acceptable carrier which is at least partially aqueous;
wherein either (i) the formulation is a hydrogel and comprises a swellable, gel-forming polymer, or (ii) the formulation is able to form a hydrogel in situ following administration, and comprises a thermo-reversible hydrogel-forming polymer; and
wherein said method comprises topically administering the formulation to any part of the oral cavity of the individual.

2. An oral delivery system for use in a method for preventing or treating an adverse oral condition in a mammalian individual susceptible to or afflicted with said adverse oral condition, said oral delivery system comprising a liposomal dispersion of the formulation defined in claim 1,
wherein said method comprises topically administering the formulation to any part of the oral cavity of the individual.

3. The formulation for use of claim 1, wherein the biocompatible chelating agent is selected from ethylenediamine tetraacetic acid (EDTA), cyclohexanediamine tetraacetic acid (CDTA), hydroxyethylethylenediamine triacetic acid (HEDTA), diethylenetriamine pentaacetic acid (DTPA), dimercaptopropane sulfonic acid (DMPS), dimercaptosuccinic acid (DMSA), aminotrimethylene phosphonic acid (ATPA), citric acid, curcumin, and acceptable salts thereof, and combinations of any of the foregoing; or is selected from chelating antibiotics, chelating agents containing two or more chelating nitrogen atoms, phosphates, and deferoxamine.

4. The formulation for use of claim 3, wherein the biocompatible chelating agent is an acceptable EDTA salt selected from diammonium EDTA, disodium EDTA, dipotassium EDTA, triammonium EDTA, trisodium EDTA, tetrasodium EDTA, tripotassium EDTA, calcium disodium EDTA, and combinations thereof.

5. The formulation for use of claim 3, wherein the chelating agent is a chelating antibiotic selected from chloroquine and tetracycline; or is selected from pyrophosphates, tripolyphosphates, hexametaphosphates, and combinations thereof.

6. The formulation for use of claim 1, wherein the biocompatible chelating agent is a sequestrant of divalent or polyvalent metal cations and represents 0.1 to 15 wt. % of the formulation.

7. The formulation for use of claim 1, wherein the methylsulfonylmethane represents approximately 0.1 wt. % to 40 wt. % of the formulation.

8. A sterile insert for delivery of a formulation to the oral cavity, comprising:
a controlled release implant housing the formulation for use of claim 1 and suitable for implantation into any part of the oral cavity,
wherein the implant is comprised of a polymeric matrix that is configured to gradually release the formulation to the oral tissues through dissolution of the matrix and/or diffusion, and that is completely soluble and/or biodegradable in the oral tissues,
wherein the implant comprises a reservoir housing the formulation and enclosed in a polymeric membrane through which the formulation gradually diffuses.

9. The insert of claim 8, wherein the insert provides for controlled release of the formulation over a time period of from 12 hours to 12 months or more following implantation of the insert into any tissue of the oral cavity.

10. The insert of claim 8, wherein the implant comprises an osmotic system from which the formulation is gradually released as a result of increased osmotic pressure within the system following implantation in the oral tissues.

11. The formulation for use of any one of claims 1 to 7, wherein the adverse oral condition: is associated with oxidative and/or free radical damage to the oral tissues; is a condition, disease, or disorder of the oral cavity; is associated with aging; is gingivitis; is periodontal disease; relates to the formation of mineral deposits, dirty teeth, calculus, or tartar; relates to the formation of bacterial biofilms, or plaque; is dental cavities; is dental caries; or relates to AIDS.

12. The formulation for use of claim 11, wherein the adverse oral condition relates to the formation of bacterial biofilms, or plaque.

## Patentansprüche

1. Orale Formulierung zur Verwendung bei einem Verfahren zur Prävention oder Behandlung eines nachteiligen oralen Zustands bei einem Säugetierindividuum, das für den nachteiligen oralen Zustand anfällig ist oder daran leidet, wobei die orale Formulierung eine orale Lösung, Suspension, Paste oder Gel ist und umfasst:
einen biokompatiblen Chelatbildner mit einer Konzentration von mindestens 0,1 Gew.-%;
eine wirksame Konzentration eines Permeationsverstärkers, der Methylsulfonylmethan ist; und
ein pharmazeutisch annehmbarer Träger, der mindestens teilweise wässrig ist;
wobei entweder (i) die Formulierung ein Hydrogel ist und ein quellfähiges, gelbildendes Polymer umfasst oder (ii) die Formulierung in der Lage ist, nach der Verabreichung ein Hydrogel in situ zu bilden, und ein thermoreversibles, hydrogelbildendes Polymer umfasst; und
wobei das Verfahren das topische Verabreichen der Formulierung an irgendeinen Teil der Mundhöhle des Individuums umfasst.

2. Orales Verabreichungssystem zur Verwendung bei einem Verfahren zur Prävention oder Behandlung eines nachteiligen oralen Zustands bei einem Säugetierindividuum, das für den nachteiligen oralen Zustand anfällig ist oder daran leidet, wobei das orale Verabreichungssystem eine liposomale Dispersion der Formulierung nach Anspruch 1 umfasst,
wobei das Verfahren das topische Verabreichen der Formulierung an irgendeinen Teil der Mundhöhle des Individuums umfasst.

3. Formulierung zur Verwendung nach Anspruch 1, wobei der biokompatible Chelatbildner ausgewählt ist aus Ethylendiamintetraessigsäure (EDTA), Cyclohexandiamintetraessigsäure (CDTA), Hydroxyethylethylendiamintriessigsäure (HEDTA), di-Ethylentriaminpentaessigsäure (DTPA), Dimercaptopropansulfonsäure (DMPS), Dimercaptobemsteinsäure (DMSA), Aminotrimethylenphosphonsäure (ATPA), Zitronensäure, Curcumin und akzeptablen Salzen davon sowie Kombinationen von irgendwelchen der Vorhergehenden; oder ausgewählt ist aus chelatbildenden Antibiotika, Chelatbildnern, die zwei oder mehr chelatbildende Stickstoffatome enthalten, Phosphaten und Deferoxamin.

4. Formulierung zur Verwendung nach Anspruch 3, wobei der biokompatible Chelatbildner ein annehmbares EDTA-Salz ist, das ausgewählt ist aus Diammonium-EDTA, Dinatrium-EDTA, Dikalium-EDTA, Triammonium-EDTA, Trinatrium-EDTA, Tetranatrium-EDTA, Trikalium-EDTA, Calciumdinatrium-EDTA und Kombinationen davon.

5. Formulierung zur Verwendung nach Anspruch 3, wobei der Chelatbildner ein chelatbildendes Antibiotikum ist, das ausgewählt ist aus Chloroquin und Tetracyclin; oder ausgewählt ist aus Pyrophosphaten, Tripolyphosphaten, Hexametaphosphaten und Kombinationen davon.

6. Formulierung zur Verwendung nach Anspruch 1, wobei der biokompatible Chelatbildner ein Maskierungsmittel aus zweiwertigen oder mehrwertigen Metallkationen ist und 0,1 bis 15 Gew.-% der Formulierung ausmacht.

7. Formulierung zur Verwendung nach Anspruch 1, wobei das Methylsulfonylmethan ungefähr 0,1 Gew.-% bis 40 Gew.-% der Formulierung ausmacht.

8. Steriler Einsatz zur Abgabe einer Formulierung in die Mundhöhle, umfassend:
ein Implantat mit kontrollierter Freigabe, das die Formulierung zur Verwendung nach Anspruch 1 aufnimmt und zur Implantation in irgendeinen Teil der Mundhöhle geeignet ist,
wobei das Implantat aus einer polymeren Matrix besteht, die konfiguriert ist, die Formulierung durch Auflösen der Matrix und/oder Diffusion allmählich an die oralen Gewebe freizugeben, und die in den oralen Geweben vollständig löslich und/oder biologisch abbaubar ist,
wobei das Implantat einen Vorratsbehälter umfasst, der die Formulierung aufnimmt und in einer Polymermembran eingeschlossen ist, durch welche die Formulierung allmählich diffundiert.

9. Einsatz nach Anspruch 8, wobei der Einsatz eine kontrollierte Freisetzung der Formulierung über einen Zeitraum von 12 Stunden bis 12 Monaten oder länger nach der Implantation des Einsatzes in irgendein Gewebe der Mundhöhle bereitstellt.

10. Einsatz nach Anspruch 8, wobei das Implantat ein osmotisches System umfasst, aus dem die Formulierung infolge eines erhöhten osmotischen Drucks innerhalb des Systems nach der Implantation in das Mundgewebe allmählich freigegeben wird.

11. Formulierung zur Verwendung nach einem der Ansprüche 1 bis 7, wobei der nachteilige orale Zustand: mit einer oxidativen Schädigung und/oder einer Schädigung durch freie Radikale am oralen Gewebe assoziiert ist; ein Zustand, eine Krankheit oder Störung der Mundhöhle ist; mit dem Altern assoziiert ist; Gingivitis ist; eine parodontale Krankheit ist; im Zusammenhang mit der Bildung von Mineralablagerungen, schmutzigen Zähnen, Zahnstein oder Zahnbelag steht; im Zusammenhang mit der Bildung von bakteriellen Biofilmen oder Plaque steht; Zahnhöhlen sind; Zahnkaries ist; oder im Zusammenhang mit AIDS steht.

12. Formulierung zur Verwendung nach Anspruch 11, wobei der nachteilige orale Zustand im Zusammenhang mit der Bildung von bakteriellen Biofilmen oder Plaque steht.

## Revendications

1. Préparation orale, pour l'utilisation dans un procédé pour la prévention ou le traitement d'une condition orale indésirable chez un individu mammifère sujet à, ou atteint de, ladite condition orale indésirable, ladite préparation orale étant une solution, suspension, pâte ou un gel oral, et comprenant :
un agent chélateur biocompatible à une concentration d'au moins 0,1 % en poids ;
une concentration efficace d'un agent améliorant la perméation, qui est du méthylsulfonylméthane ; et
un vecteur pharmaceutiquement acceptable qui est au moins partiellement aqueux ; dans laquelle (i) la préparation est un hydrogel et comprend un polymère gélifiant gonflant, ou (ii) la préparation est capable de former un hydrogel in situ suivant l'administration, et comprend un polymère hydrogélifiant thermoréversible ; et
dans laquelle ledit procédé comprend l'administration topique de la préparation à une partie quelconque de la cavité orale de l'individu.

2. Système d'administration orale pour l'utilisation dans un procédé pour la prévention ou le traitement d'une condition orale indésirable chez un individu mammifère sujet à, ou atteint de, ladite condition orale indésirable, ledit système d'administration orale comprenant une dispersion liposome de la préparation définie dans la revendication 1,
dans lequel ledit procédé comprend l'administration topique de la préparation à une partie quelconque de la cavité orale de l'individu.

3. Préparation pour l'utilisation selon la revendication 1, dans laquelle l'agent chélateur biocompatible est sélectionné parmi acide éthylène-diamine tétra-acétique (EDTA), acide cyclohexane-diamine tétra-acétique (CDTA), acide hydroxyéthyle-éthylène-diamine tri-acétique (HEDTA), acide diéthylène-triamine penta-acétique (DTPA), acide dimercaptopropane-sulfonique (DMPS), acide dimercapto-succinique (DMSA), acide aminotriméthylène-phosphonique (ATPA), acide citrique, curcumine, et des sels acceptables de ceux-ci, et des combinaisons de quelconques de ce qui précède ; ou est sélectionné parmi des antibiotiques chélateurs, des agents chélateurs contenant deux, ou plus, atomes d'azote chélateurs, des phosphates, et de la déféroxamine.

4. Préparation pour l'utilisation selon la revendication 3, dans laquelle l'agent chélateur biocompatible est un sel d'EDTA acceptable sélectionné parmi EDTA de diammonium, EDTA de disodium, EDTA de dipotassium, EDTA de triammonium, EDTA de trisodium, EDTA de tétrasodium, EDTA de tripotassium, EDTA de calcium-disodium, et des combinaisons de ceux-ci.

5. Préparation pour l'utilisation selon la revendication 3, dans laquelle l'agent chélateur est un antibiotique chélateur sélectionné parmi la chloroquine et la tétracycline ; ou est sélectionné parmi les pyrophosphates, tripolyphosphates, hexamétaphosphates, et des combinaisons de ceux-ci.

6. Préparation pour l'utilisation selon la revendication 1, dans laquelle l'agent chélateur biocompatible est un séquestrant de cations de métal divalent ou polyvalent et représente de 0,1 à 15 % en poids de la préparation.

7. Préparation pour l'utilisation selon la revendication 1, dans laquelle le méthylsulfonylméthane représente approximativement de 0,1 % en poids à 40 % en poids de la préparation.

8. Élément à insérer stérile pour l'administration d'une formulation à la cavité orale, comprenant :
un implant à libération contrôlée contenant la préparation pour l'utilisation de la revendication 1 et approprié pour l'implantation dans une partie quelconque de la cavité orale,
dans lequel l'implant est composé d'une matrice polymère qui est configurée pour progressivement libérer la préparation aux tissus oraux par l'intermédiaire de dissolution de la matrice et/ou de diffusion, et qui est complètement soluble et/ou biodégradable dans les tissus oraux,
dans lequel l'implant comprend un réservoir contenant la préparation et enfermé dans une membrane polymère à travers laquelle la préparation se diffuse progressivement.

9. Élément à insérer selon la revendication 8, dans lequel l'élément à insérer permet la libération contrôlée de la préparation durant une période de 12 heures à 12 mois ou plus suivant l'implantation de l'élément à insérer dans un tissu quelconque de la cavité orale.

10. Élément à insérer selon la revendication 8, dans lequel l'implant comprend un système osmotique à partir duquel la préparation est progressivement libérée en conséquence de pression osmotique accrue à l'intérieur du système suivant l'implantation dans les tissus oraux.

11. Préparation pour l'utilisation selon l'une quelconque des revendications 1 à 7, dans laquelle la condition orale indésirable : est associée à une lésion oxydative et/ou due à des radicaux libres des tissus oraux ; est une condition, une maladie, ou un trouble de la cavité orale ; est associée au vieillissement ; est la gingivite ; est une maladie parodontale ; est connexe à la formation de dépôts minéraux, de dents sales, de calcul, ou de tartre ; est connexe à la formation de biofilms bactériens, ou de plaque ; est composée de cavités dentaires ; est composée de caries dentaires ; ou est connexe au SIDA.

12. Préparation pour l'utilisation de la revendication 11, dans laquelle la condition orale indésirable est connexe à la formation de biofilms bactériens, ou de plaque.
